# EUROPEAN PATENT APPLICATION

(11) **EP 1 709 973 A1**
(43) Date of publication of application: **11.10.2006**
(21) Application number: 04808150.9
(22) Date of filing: 24.12.2004
(51) Int. Cl.: A61K 45/00, A61K 48/00, A61K 31/7088, A61K 41/00, A61P 35/00, A61P 35/02, A61P 35/04

(54) **METHOD OF SUPPRESSING CANCER**

(30) Priority: 24.12.2003 JP 2003428300
(71) Applicant: Locomogene, Inc., Tokyo 105-0001 (JP)
(72) Inventor: NAKAJIMA, Toshihiro;, u, Yokohama, Kanagawa; 2240001 (JP); YAMASAKI, Satoshi;, ba-ku, Yokohama, Kanagawa; 2250003 (JP); YAGISHITA, Naoko;, okohama, Kanagawa; 2340053 (JP)
(74) Representative: Woods, Geoffrey Corlett
(86) International application number: PCT/JP2004/019800
(87) International publication number: WO 2005/061001

(57) **Abstract**

The present invention provides a method for suppressing cancer comprising activating tumor suppressor gene p53 or protein p53 for localizing the protein to the nuclear, and a pharmaceutical composition comprising a substance that promotes activation of tumor suppressor gene p53 or protein p53.

SEQUENCE LISTING

<110>  LOCOMOGENE, INC.

<120>  A METHOD OF INHIBITING A CANCER

<130>  N.98616

<140>  EP04808150.9
<141>  2004-12-24

<150>  PCT/JP2004/19800
<151>  2004-12-24

<150>  JP2003-428300
<151>  2003-12-24

<160>  19

<170>  PatentIn version 3.2

<210>  1
<211>  3374
<212>  DNA
<213>  Homo sapiens

<220>
<221>  CDS
<222>  (403)..(2256)
<223>

<400>  1
gccctttctt atgagcatgc ctgtgttggg ttgacagtga gggtaataat gacttgttgg      60
ttgattgtag atatagggct ctcccttgca aggtaattag gctccttaaa ttacctgtaa     120
gattttcttg ccacagcatc cattctggtt aggctggtga tcttctgagt agtgatagat     180
tggttggtgg tgaggtttac aggtgttccc ttctcttact cctggtgttg gctacaatca     240
ggtggcgtct agagcagcat gggacaggtg ggtaagggga gtcttctcat tatgcagaag     300
tgatcaactt aaatctctgt cagatctacc tttatgtagc ccggcagtcg cgcggattga     360
gcgggctcgc ggcgctgggt tcctggtctc cgggccaggg ca atg ttc cgc acg        414
gca gtg atg atg gcg gcc agc ctg gcg ctg acc ggg gct gtg gtg gct       462
cac gcc tac tac ctc aaa cac cag ttc tac ccc act gtg gtg tac ctg       510
acc aag tcc agc ccc agc atg gca gtc ctg tac atc cag gcc ttt gtc       558
ctt gtc ttc ctt ctg ggc aag gtg atg ggc aag gtg ttc ttt ggg caa       606
ctg agg gca gca gag atg gag cac ctt ctg gaa cgt tcc tgg tac gcc      654
gtc aca gag act tgt ctg gcc ttc acc gtt ttt cgg gat gac ttc agc      702
ccc cgc ttt gtt gca ctc ttc act ctt ctt ctc ttc ctc aaa tgt ttc      750
cac tgg ctg gct gag gac cgt gtg gac ttt atg gaa cgc agc ccc aac      798
atc tcc tgg ctc ttt cac tgc cgc att gtc tct ctt atg ttc ctc ctg      846
ggc atc ctg gac ttc ctc ttc gtc agc cac gcc tat cac agc atc ctg      894
acc cgt ggg gcc tct gtg cag ctg gtg ttt ggc ttt gag tat gcc atc      942
ctg atg acg atg gtg ctc acc atc ttc atc aag tat gtg ctg cac tcc      990
gtg gac ctc cag agt gag aac ccc tgg gac aac aag gct gtg tac atg     1038
ctc tac aca gag ctg ttt aca ggc ttc atc aag gtt ctg ctg tac atg     1086
gcc ttc atg acc atc atg atc aag gtg cac acc ttc cca ctc ttt gcc     1134
atc cgg ccc atg tac ctg gcc atg aga cag ttc aag aaa gct gtg aca     1182
gat gcc atc atg tct cgc cga gcc atc cgc aac atg aac acc ctg tat     1230
cca gat gcc acc cca gag gag ctc cag gca atg gac aat gtc tgc atc     1278
atc tgc cga gaa gag atg gtg act ggt gcc aag aga ctg ccc tgc aac     1326
cac att ttc cat acc agc tgc ctg cgc tcc tgg ttc cag cgg cag cag      1374
acc tgc ccc acc tgc cgt atg gat gtc ctt cgt gca tcg ctg cca gcg      1422
cag tca cca cca ccc ccg gag cct gcg gat cag ggg cca ccc cct gcc      1470
ccc cac ccc cca cca ctc ttg cct cag ccc ccc aac ttc ccc cag ggc      1518
ctc ctg cct cct ttt cct cca ggc atg ttc cca ctg tgg ccc ccc atg      1566
ggc ccc ttt cca cct gtc ccg cct ccc ccc agc tca gga gag gct gtg      1614
gct cct cca tcc acc agt gca gca gcc ctt tct cgg ccc agt gga gca      1662
gct aca acc aca gct gct ggc acc agt gct act gct gct tct gcc aca      1710
gca tct ggc cca ggc tct ggc tct gcc cca gag gct ggc cct gcc cct      1758
ggt ttc ccc ttc cct cct ccc tgg atg ggt atg ccc ctg cct cca ccc      1806
ttt gcc ttc ccc cca atg cct gtg ccc cct gcg ggc ttt gct ggg ctg      1854
acc cca gag gag cta cga gct ctg gag ggc cat gag cgg cag cac ctg      1902
gag gcc cgg ctg cag agc ctg cgt aac atc cac aca ctg ctg gac gcc      1950
gcc atg ctg cag atc aac cag tac ctc acc gtg ctg gcc tcc ttg ggg      1998
ccc ccc cgg cct gcc act tca gtc aac tcc act gag ggg act gcc act      2046
aca gtt gtt gct gct gcc tcc tcc acc agc atc cct agc tca gag gcc 2094
acg acc cca acc cca gga gcc tcc cca cca gcc cct gaa atg gaa agg 2142
cct cca gct cct gag tca gtg ggc aca gag gag atg cct gag gat gga 2190
gag ccc gat gca gca gag ctc cgc cgg cgc cgc ctg cag aag ctg gag 2238
tct cct gtt gcc cac tga cactgcccca gcccagcccc agcctctgct 2286
cttttgagca gccctcgctg gaacatgtcc tgccaccaag tgccagctcc ctctctgtct 2346
gcaccaggga gtagtacccc cagctctgag aaagaggcgg catcccctag gccaagtgga 2406
aagaggctgg ggttcccatt tgactccagt cccaggcagc catggggatc tcgggtcagt 2466
tccagccttc ctctccaact cttcagccct gtgttctgct ggggccatga aggcagaagg 2526
tttagcctct gagaagccct cttcttcccc cacccctttc caggagaagg ggctgcccct 2586
ccaagcccta cttgtatgtg cggagtcaca ctgcagtgcc gaacagtatt agctcccgtt 2646
cccaagtgtg gactccagag gggctggagg caagctatga acttgctcgc tggcccaccc 2706
ctaagactgg tacccatttc cttttcttac cctgatctcc ccagaagcct cttgtggtgg 2766
tggctgtgcc ccctatgccc tgtggcattt ctgcgtctta ctggcaacca cacaactcag 2826
ggaaaggaat gcctgggagt gggggtgcag gcgggcagca ctgagggacc ctgccccgcc 2886
cctcccccca ggcccctttc ccctgcagct tctcaagtga gactgacctg tctcacccag 2946
cagccactgc ccagccgcac tccaggcaag ggccagtgcg cctgctcctg accactgcaa 3006
tcccagcgcc caaggaaggc cacttctcaa ctggcagaac ttctgaagtt tagaattgga 3066
attacttcct tactagtgtc ttttggctta aattttgtct tttgaagttg aatgcttaat 3126
cccgggaaag aggaacagga gtgccagact cctggtcttt ccagtttaga aaaggctctg 3186
tgccaaggag ggaccacagg agctgggacc tgcctgcccc tgtcctttcc ccttggtttt 3246
gtgttacaag agttgttgga gacagtttca gatgattatt taatttgtaa atattgtaca 3306
aattttaata gcttaaattg tatatacagc caaataaaaa cttgcattaa caaaaaaaaa 3366
aaaaaaaa 3374

<210> 2

<211>  617
<212>  PRT
<213>  Homo sapiens

<400>  2

<210>  3
<211>  23
<212>  DNA
<213>  Homo sapiens

<400>  3
aatgtctgca tcatctgccg aga                                              23

<210>  4
<211>  23
<212>  DNA
<213>  Homo sapiens

<400>  4
aagctgtgac agatgccatc atg                                              23

<210>  5
<211>  23
<212>  DNA

<213>  Homo sapiens

<400>  5
aaagctgtga cagatgccat cat                                              23


<210>  6
<211>  23
<212>  DNA
<213>  Homo sapiens

<400>  6
aagaaagctg tgacagatgc cat                                              23


<210>  7
<211>  23
<212>  DNA
<213>  Homo sapiens

<400>  7
aaggttctgc tgtacatggc ctt                                              23


<210>  8
<211>  23
<212>  DNA
<213>  Homo sapiens

<400>  8
aacaaggctg tgtacatgct cta                                              23


<210>  9
<211>  23
<212>  DNA
<213>  Homo sapiens

<400>  9
aaatgtttcc actggctggc tga                                              23


<210>  10
<211>  23
<212>  DNA
<213>  Homo sapiens

<400>  10
aaggtgttct ttgggcaact gag                                              23


<210>  11
<211>  23
<212>  DNA
<213>  Homo sapiens

<400>  11
aacatccaca cactgctgga cgc                                              23


<210>  12

<211>  23
<212>  DNA
<213>  Homo sapiens

<400>  12
aacaccctgt atccagatgc cac                                              23


<210>  13
<211>  23
<212>  DNA
<213>  Homo sapiens

<400>  13
aaggtgcaca ccttcccact ctt                                              23


<210>  14
<211>  23
<212>  DNA
<213>  Homo sapiens

<400>  14
aatgtttcca ctggctggct gag                                              23


<210>  15
<211>  23
<212>  DNA
<213>  Homo sapiens

<400>  15
aagagactgc cctgcaacca cat                                              23


<210>  16
<211>  23
<212>  DNA
<213>  Homo sapiens

<400>  16
aacgttcctg gtacgccgtc aca                                              23


<210>  17
<211>  393
<212>  PRT
<213>  Homo sapiens

<400>  17

<210>  18
<211>  21
<212>  DNA
<213>  Artificial sequence

<220>
<223>  synthetic oligonucleotide (DNA/RNA mixture)

<400>  18
gguguucuuu gggcaacugt t                                                21

<210>  19
<211>  21
<212>  DNA
<213>  Artificial sequence

<220>
<223>  synthetic oligonucleotide (DNA/RNA mixture)

<400>  19
caguugccca aagaacacct t                                                21

## Description

### FIELD OF THE INVENTION

The present invention relates to a method for activating tumor suppressor gene p53 or protein p53 for localizing protein p53 to the nuclear. Furthermore, the present invention relates to a pharmaceutical composition comprising a substance that promotes activation of tumor suppressor gene p53 or protein p53.

### BACKGROUND OF THE INVENTION

Synoviolin is a novel protein found as a membrane protein overexpressed in synovial cells derived from patients suffering from rheumatism (WO 02/052007). From studies using genetically modified animals, synoviolin was found to be an essential molecule for onset of rheumatoid arthritis.

Protein structure prediction system suggests that synoviolin has a RING finger motif. This motif is often found in an enzyme called ubiquitin ligase E3 that plays an important role in protein ubiquitination. In fact, synoviolin is proved to have autoubiquitination activity, which is one of the characteristics of ubiquitin ligase E3 (WO 02/052007).

On the other hand, gene p53 residing on chromosome 17p13 is a tumor suppressor gene critical for development and growth of tumor cells. Protein p53 recognizes a specific nucleotide sequence [5'-(A/T)GPyPyPy-3'] on DNA, and promotes transcription activation of specific genes such as waf1/cip1, GADD45 and BAX. Moreover, protein p53 is known for its physiologic functions such as (i) function of suppressing transcriptions of numbers of other genes; (ii) function of binding to viral oncogenes such as SV40 large T antigen, adenovirus EIB protein and papillomavirus E6 or cellular oncogenes such as mdm2; and (iii) function of specifically binding to DNA containing mismatch.

Thus, in order to find a cancer suppressing substance, it is important to analyze molecules that control functions of tumor suppressor gene p53 or protein p53.

### DISCLOSURE OF THE INVENTION

An objective of the present invention is to provide a method for promoting activation of tumor suppressor gene p53 or protein p53, and to provide a pharmaceutical composition that promotes activation of tumor suppressor gene p53 or protein p53.

We have gone through keen study to solve the above problem. In detailed analysis of a synoviolin homozygous knockout animal, a significant number of apoptotic cells were observed as compared to a wild type while protein p53 that is closely related to apoptosis induction was found strongly expressed and localized to the nuclei. We found that inhibition of synoviolin function activates tumor suppressor gene p53 or tumor suppressor protein p53 that inhibits growth of tumor cells, based on which we accomplished the present invention.

Thus, the present invention is as follows.
(1) A pharmaceutical composition comprising a substance that promotes activation of tumor suppressor gene p53 or activity of protein p53.
(2) A pharmaceutical composition according to (1), wherein the substance that promotes activation of tumor suppressor gene p53 or protein p53 is a substance that inhibits synoviolin expression and/or function.
(3) A pharmaceutical composition according to (2), wherein the substance that inhibits synoviolin expression and/or function is siRNA or shRNA that targets a gene coding for synoviolin.
(4) A pharmaceutical composition according to (3), wherein the gene coding for synoviolin comprises the nucleotide sequence represented by SEQ ID NO: 1.
(5) A pharmaceutical composition according to (3), wherein siRNA targets a part of the nucleotide sequence represented by SEQ ID NO: 1.
(6) A pharmaceutical composition according to any one of (1) to (5) for treating cancer.
(7) A method for activating tumor suppressor gene p53 or protein p53, comprising inhibiting synoviolin expression and/or function.
(8) A method for localizing protein p53 to the nucleus, comprising inhibiting synoviolin expression and/or function.
(9) A method for suppressing cancer, comprising inhibiting synoviolin expression and/or function to localize protein p53 to the nucleus.
(10) A method according to (9) further comprising irradiating protein p53 localized to the nucleus with radiation or ultraviolet.
(11) A method according to (9) further comprising contacting a cell containing protein p53 localized to the nucleus with an anticancer agent, or further comprising embolizing a vessel around said cell.
(12) A method for phosphorylating a part of amino acid residues of protein p53, comprising inhibiting synoviolin expression and/or function.
(13) A method according to (12) wherein the part of amino acid residues is serine residue at position 15.
(14) A method for activating kinase comprising inhibiting synoviolin expression and/or function.
(15) A method according to (14) wherein the kinase comprises ATM, ATR or an enzyme having a similar activity thereto.
(16) A method for inducing expression of protein p21 with activated protein p53, comprising inhibiting synoviolin expression and/or function to activate p53 protein.
(17) A method for suppressing cancer comprising inhibiting synoviolin expression and/or function to allow protein p53 to induce expression of protein p21.
(18) A method for inhibiting protein p53 ubiquitination comprising inhibiting synoviolin expression and/or function.
(19) A method according to any one of (7) to (18) wherein the synoviolin expression is inhibited with siRNA or shRNA that targets a gene coding for synoviolin.
(20) A method according to any one of (7) to (18) wherein the synoviolin function is inhibited by inhibiting a function of synoviolin to bind to protein p53.
(21) A method according to (19) wherein the gene coding for synoviolin comprises the nucleotide sequence represented by SEQ ID NO: 1.
(22) A method according to (19) wherein siRNA targets a part of the nucleotide sequence represented by SEQ ID NO: 1.

### BRIEF DESCRIPTION OF THE DRAWINGS

Figure 1 are pictures showing the results of immunohistostaining in synoviolin homozygous knockout fetal mouse fibroblasts (MEFs).
Figure 2 are pictures showing the results of immunohistostaining with an anti-p53 antibody in syno-/- embryo.
Figure 3 are pictures showing the results of western blotting for p53.
Figure 4 are pictures showing the results of identification of a phosphorylated site of p53 in syno-/- MEF cultured cells.
Figure 5 is a picture of western blotting for examining how Ser15 phosphorylation enhanced by siRNA treatment targeting synoviolin is affected by addition of caffeine.
Figure 6 are pictures of western blotting showing that siRNA treatment targeting synoviolin enhances p53 and p21 expressions.
Figure 7 are diagrams showing cell cycles observed with a flow cytometer.
Figure 8 are pictures showing the results of immunostaining of Tissue arrays using an anti-synoviolin antibody (10 Da).
Figure 9 are pictures showing the results of immunostaining of Tissue arrays using an anti-synoviolin antibody (10 Da).
Figure 10 are pictures for monitoring p53 localization in cells transfected with GFP wild-type p53.
Figure 11 are pictures for monitoring p53 localization by co-expressing GFP wild-type p53 and FLAG wild-type synoviolin and staining the nuclei with 400-fold diluted primary antibody α-FLAG antibody, 200-fold diluted secondary antibody α-mouse IgG-TRITC or 1 µM DAPI.
Figure 12 are pictures for monitoring p53 localization by co-expressing GFP wild □type p53 and FLAG wild-type synoviolin C307S and staining the nuclei with 400-fold diluted primary antibody α-FLAG antibody, 200-fold diluted secondary antibody α-mouse IgG-TRITC or 1 µM DAPI.
Figure 13 is a picture showing *in vitro* ubiquitination reaction of GST p53 with MBP-synoviolin dTM-His.
Figure 14 is a graph showing the amounts of p53 mRNA in RA synovial cells upon synoviolin RNAi.
Figure 15 is a schematic diagram of prepared mutants lacking p53-binding domains and the results of binding assays.
Figure 16 is a view showing the results of GST pulldown assays for the mutants lacking p53-binding domains and ³⁵S-p53.

### BEST MODES FOR CARRYING OUT THE INVENTION

Hereinafter, the present invention will be described in details.

The present invention is characterized by inhibiting synoviolin expression and/or function to localize p53 (referring to tumor suppressor gene p53 or protein p53) to the nucleus and to activate it, thereby suppressing cancer. The present invention was accomplished based on the finding that inhibition of synoviolin expression and/or function allows kinase to phosphorylate and activate p53 that enhances expression of p21, a cyclin-dependent kinase inhibitor, consequently suppressing development or growth of cancer by preventing G1 to S phase progression in a tumor cell and the finding that synoviolin suppresses p53 expression via a ubiquitin ligase thereof.

### 1. p53 Activation

### (1) Inhibition of synoviolin expression and/or function, and activation of p53

Since exposure of a normal cell to ultraviolet or the like activate p53, and which arrests cell cycle and stabilization, increasing the concentration of p53 can stop growth of a tumor cell. In other words, if p53 is not working, tumor cell growth is not prevented and hence cancer progresses. In fact, p53-defective mutation is rarely observed in cells of a normal individual while the p53-defective mutation is caused in about half the cells derived from a cancer patient. Even when such mutation is absent, some kind of mutation is caused in the p53-controlling mechanism, thereby deteriorating the cancer-suppressing function. Therefore, it is necessary to allow p53 to function effectively for preventing the progression of cancer.

According to the present invention, we focused attention on synoviolin function for utilizing p53 activation as an effective method for cancer treatment. A synoviolin homozygous knockout animal was prepared for detailed analysis, in which a significant number of apoptotic cells were observed as compared to a wild-type animal. Specifically, we found that inhibition of synoviolin function promotes activation of p53 that is closely related to apoptosis, and hence inhibition of synoviolin function results in cancer suppression.

The phrase "synoviolin expression" as used herein means transcription and translation of a gene coding for synoviolin or production of synoviolin protein resulting from these transcription and translation. The phrase "synoviolin function" means suppression of p53 activation, including functions of synoviolin to bind to p53 and to ubiquitinate p53. Therefore, the phrase "to inhibit synoviolin expression and/or function" refers to decreasing or eliminating the amount, function or activity of synoviolin gene or protein compared to wild-type gene or protein. The term "inhibition" includes inhibition of both function and expression, and inhibition of either the function or the expression.

Since synoviolin promotes ubiquitination of p53, inhibition of binding between synoviolin and p53 can inhibit p53 ubiquitination, allowing p53 to activate and result in cancer suppression.

Apoptosis means a programmed cell death caused by the cell itself, characterized by chromosome condensation in the cell nucleus, fragmentation of the cell nucleus, loss of cell surface microvilli, cell aggregation, caspase activation and loss of mitochondrial membrane potential. Apoptosis is considered to have taken place when the above characteristics are caused in the cell.

According to the present invention, immunostaining of p53 in a fetal embryo shows strong expression of p53 throughout the synoviolin homozygous knockout fetal mouse embryo. Fetal fibroblasts (MEFs) isolated from the synoviolin homozygous knockout fetal mouse embryo also showed strong expression compared to those isolated from a wild type and p53 was strongly located to the nucleus. Such localization was not observed in the wild-type nucleus. When synoviolin and p53 are allowed to express strongly, p53 co-localizes with synoviolin in the cytoplasm. This means that inhibition of synoviolin expression and/or function allows translocation of p53 to the nuclear. Furthermore, synoviolin homozygous knockout fetal mouse MEFs show high radiosensitivity and ultraviolet sensitivity. Thus, according to the present invention, inhibition of synoviolin expression and/or function in a tumor cell for translocating p53 to the nucleus of the tumor cell, and subsequent radiation or ultraviolet irradiation of the p53 can effectively suppress propagation of tumor cell. Means for radiating radiation is not particularly limited and, for example, gamma ray of 1 to 10 Gy can be radiated. Ultraviolet irradiation can be carried out by radiating ultraviolet (wavelength 100 to 400 nm, preferably 290 to 400 nm) using an appropriate ultraviolet irradiation apparatus (available from Funakoshi, Dermaray, Keyence, etc.).

Furthermore, the present invention can efficiently suppress cancer by further bringing a cell (in particular a tumor cell) containing p53 localized to the nucleus into contact with an anticancer agent. Alternatively, embolization of a vessel (e.g., blood vessel or lymph vessel) around a tumor cell containing p53 located to the nucleus can also suppress cancer.

Examples of "anticancer agents" include alkylating agents, antimetabolites, microtubule inhibitors, platinum complex compounds and molecular target agents.
Specific examples of these anticancer agents include but not limited to the followings:
<Alkylating drugs>
Mustards: cyclophosphoamide (endoxan), merphalan, etc.
Aziridines: thiotepa, etc.
Alkyl sulfones: busulfan, etc.
Nitrosoureas: nimustine, lomustine, etc.
<Antimetabolites>
Folate derivatives: methotrexate, etc.
Purine derivatives: mercaptopurine, azathioprine, etc.
Pyrimidine derivatives: 5-fluorouracil, tegafur, carmofur, etc.
<Microtubule inhibitors>
Vinca alkaloids: vincristine, vinblastine, etc.
Taxane: paclitaxel, docetaxel, etc.
<Hormone analogs>
Tamoxifen, estrogen, etc.
<Platinum complex compounds>
Cisplatin, carboplatin, etc.
<Molecular target agents>
Imatinib, rituximab, gefitinib, etc.

A method for bringing a tumor cell into contact with an anticancer agent may be such that the anticancer agent is added to a cell or a tissue (a cancer cell or a cancer tissue) including a cell with p53 located to the nucleus, or such that the anticancer agent is administered to a tumor-bearing patient or a tumor-bearing animal. The amount of anticancer agent used may be but not limited to 100 pM to 100 µM, preferably 1 nM to 10 µM, where the anticancer agent is added. The dose for administration is 0.1 to 100 mg/kg/day, preferably 2 to 25 mg/kg/day, when using endoxan as the anticancer agent. Those skilled in the art can appropriately determine dose or amount for anticancer agents other than endoxan.

For embolization of a vessel around a cancer cell containing p53 located to the nucleus, a thrombus may be formed in blood vessels around a cell population or a tissue including the cancer cell with p53 located to the nucleus while fat, air or gas embolization may be performed on blood vessels or lymph vessels.

### (2) Promotion of p53 phosphorylation and p53 activation by inhibition of synoviolin expression and/or function

Furthermore, according to the present invention, a part of p53 amino acid residues is phosphorylated for p53 activation. The amino acid residue to be phosphorylated for p53 activation is preferably a serine residue of the p53 amino acid sequence, more preferably serine residue at position 15 (Ser15).

Phosphorylation of Ser15 of p53 enhances p53 expression and transcription activity, and therefore increases the number of transcripts. Kinases such as ATM (ataxia-telangiectasia mutated) and ATR (ataxia-telangiectasia related) are closely related to the Ser15 phosphorylation of p53. ATM is a causative protein of ataxia telangiectasia (a human autosomal recessive genetic disease), which has a function of controlling propagation of the cell by sensing DNA lesion and phosphorylating tumor suppressor gene p53. ATR, a member of the ATM family, is a kinase derived with a wide range of chemotherapeutic agents, ultraviolet irradiation or protein kinase inhibition, and involved in p53 activation that does not involve ATM.

Caffeine is known to inhibit ATM and ATR functions and thus was used in an experiment according to the present invention in which synoviolin expression and/or function was inhibited to confirm that synoviolin regulated ATM and ATR activation.

Specifically, when synoviolin expression and/or function are inhibited in the absence of caffeine, p53 is activated. On the other hand, when synoviolin expression and/or function are inhibited in the presence of caffeine, p53 activation is suppressed. It is also known that caffeine inhibits ATM and ATR activities (p53 phosphorylation).

Since p53 is not activated when ATM and ATR is inhibited with caffeine even though synoviolin expression and/or function are inhibited, a conceivable mechanism is that inhibition of synoviolin expression and/or function activates ATM and ATR that induce p53 activation. In this case, inhibition of synoviolin expression and/or function should increase the activities of these kinases. Therefore, the present invention is characterized by inhibiting synoviolin expression and/or function for promoting kinase activation. Enzymes having similar activities to ATM and ATR (enzymes that phosphorylates p53) may be, for example, DNA-PK or GSK3β.

Protein p21 is known as a substance whose expression is induced by phosphorylation of Ser15 of p53. Protein p21 acts as an inhibitor for cyclin-dependent kinase (CDK) activity and controls a cell cycle through inhibition of the CDK activity. CDK is a key of a cell cycle control and works with its partner, i.e., a cyclin protein, for example, to control smooth progression from G1 phase during which the cell is at rest to S phase during which DNA is replicated. In a cancer cell, p53 activation enhances expression of p21 as a CDK inhibitor, which in turn inhibits progression from G1 phase to S phase in the tumor cell, thereby suppressing the cancer. Thus, the present invention is characterized by inhibiting synoviolin expression and/or function as described above to enhance p53 activity and induce p21 expression to inhibit CDK, thereby suppressing cancer.

### (3) Inhibition of p53 ubiquitination and p53 stabilization

Synoviolin ubiquitinates p53. Ubiquitination refers to a post-translation modification reaction of a protein with ubiquitin, i.e., a protein degradation marker molecule. The physiologic significance of ubiquitination has conventionally been recognized for tag modification that directs to the proteosome proteolytic system. According to subsequent studies, significance of ubiquitination as of today is characterized by a reversible protein modification system that controls protein functions.

Specifically, ubiquitination repeats cascade reactions in which enzymes such as ubiquitin-activating enzyme (E1), ubiquitin-conjugating enzyme (E2) and ubiquitin ligase (E3) cooperate to conjugate ubiquitin molecules to a substrate protein in a branching manner to form a polyubiquitin chain. This polyubiquitin chain is formed via ε-amino group of the lysine residue at position 48 in ubiquitin molecule and serves as a degradation signal to 26S proteasome resulting in degradation of the target protein.

The present invention is characterized by inhibiting synoviolin expression and/or function to activate p53. Such p53 activation is based on inhibition of p53 ubiquitination apart from the p53 phosphorylation described above.

### (4) Determination of binding site between synoviolin and p53

A p53-binding site in synoviolin can be determined by preparing different types of mutants lacking p53-binding domains by deleting certain domains of the synoviolin amino acid sequence for performing GST pulldown assay for ³⁵S-p53. Specifically, the synoviolin mutants lacking p53-binding domains are expressed in *E. coli* or the like as GST-fusion proteins to confirm protein-protein bindings with protein ³⁵S-p53 by GST pulldown assay technique.

This revealed that p53-binding domain in synoviolin was 35 amino acid residues residing at positions 236-270 of the amino acid sequence (SEQ ID NO:2) in synoviolin protein.

As described above, synoviolin promotes p53 ubiquitination. Therefore, inhibition of one of the synoviolin functions, i.e., p53-binding function, inhibits p53 ubiquitination, leading to p53 activation. Preferably, the domain of synoviolin protein involved in p53 binding is amino acids 236-270 in the amino acid sequence of synoviolin. Therefore, it is preferable to mainly select this domain as the target region for binding inhibition. In order to inhibit binding between synoviolin and p53, for example, a synoviolin antagonist (a low molecular compound, peptide or the like) may be used and then inhibition can be assessed by binding assay, yeast two-hybrid assay or ubiquitination activity assay. Alternatively, an antibody that recognizes the domain 236-270 in synoviolin may be reacted with synoviolin. These methods allow inhibition of binding between synoviolin and p53.

### 2. Inhibition of synoviolin expression and/or function and inhibition of activity

In order to activate p53, a method is employed that inhibits synoviolin expression and/or function.

For inhibition of synoviolin expression and/or function, for example and not by limitation, RNA interference (RNAi) may be utilized. siRNA (small interfering RNA) targeting synoviolin gene can be designed and synthesized for transduction of cells for RNAi.

RNAi is a phenomenon in which dsRNA (double-strand RNA) specifically and selectively binds to a target gene, which is subsequently removed to efficiently inhibit the expression of the target. For example, when dsRNA is introduced into a cell, expression of a gene having a homologous sequence to the RNA is knocked down.

siRNA is designed as follows.
(a) There is no limitation to the gene as long as the gene codes for synoviolin and any domains can be used as candidates. For example, in the case of human, any domains in GenBank Accession number AB024690 (SEQ ID NO:1) can be used as candidates.
(b) From the selected domains, sequences starting with AA with a length of 19 to 25 bases, preferably 19 to 21 bases are selected. The GC contents of the sequences are, for example, conveniently 40-60%. Specifically, DNA including at least one selected from the following nucleotide sequences of the nucleotide sequence represented by SEQ ID NO:1 can be used as a target sequence of siRNA. In particular, (i) SEQ ID NO: 3, (ii) SEQ ID NO: 4, (vi) SEQ ID NO: 8, (vii) SEQ ID NO: 9 and (viii) SEQ ID NO: 10 are preferable as targets.
   (i) AA TGTCTGCATCATCTGCCGA GA (SEQ ID NO:3)
   (ii) AA GCTGTGACAGATGCCATCA TG (SEQ ID NO:4)
   (iii) AA AGCTGTGACAGATGCCATC AT (SEQ ID NO:5)
   (iv) AA GAAAGCTGTGACAGATGCC AT (SEQ ID NO:6)
   (v) AA GGTTCTGCTGTACATGGCC TT (SEQ ID NO:7)
   (vi) AA CAAGGCTGTGTACATGCTC TA (SEQ ID NO:8)
   (vii) AA ATGTTTCCACTGGCTGGCT GA (SEQ ID NO:9)
   (viii) AA GGTGTTCTTTGGGCAACTG AG (SEQ ID NO:10)
   (ix) AA CATCCACACACTGCTGGAC GC (SEQ ID NO:11)
   (x) AA CACCCTGTATCCAGATGCC AC (SEQ ID NO:12)
   (xi) AA GGTGCACACCTTCCCACTC TT (SEQ ID NO:13)
   (xii) AA TGTTTCCACTGGCTGGCTG AG (SEQ ID NO:14)
   (xiii) AA GAGACTGCCCTGCAACCAC AT (SEQ ID NO:15)
   (xiv) AA CGTTCCTGGTACGCCGTCA CA (SEQ ID NO:16)
   siRNA can be introduced into a cell by a method in which siRNA synthesized *in vitro* is linked to plasmid DNA and then introduced into the cell or a method in which two RNAs are annealed.

Moreover, according to the present invention, shRNA may be used for providing RNAi effect. shRNA is an RNA molecule called short hairpin RNA that has a stem-loop structure for forming a complementary strand between one domain and the other domain of the single-stranded molecule.

shRNA can be designed such that a part thereof forms a stem-loop. For example, when sequence A represents a sequence of one domain and sequence B represents a sequence complementary to sequence A, sequence A, a spacer and sequence B are provided in this order in one RNA strand with the whole length being 45 to 60 bases. Sequence A is a part of the target synoviolin gene (SEQ ID NO:1). The target domain is not particularly limited and any domain can be a candidate. The length of sequence A is 19 to 25 bases, preferably 19 to 21 bases.

### 3. Pharmaceutical composition

shRNA and siRNA prepared according to the present invention are substances that inhibit synoviolin expression and/or function and thus may be used as a pharmaceutical composition for activating p53 (especially as a gene therapeutic agent).

The sites of application for the pharmaceutical composition of the invention as a gene therapeutic agent include but not limited to brain tumor, tongue cancer, pharynx cancer, lung cancer, breast cancer, esophageal cancer, gastric cancer, pancreas cancer, biliary cancer, gallbladder cancer, duodenal cancer, colon cancer, liver cancer, uterus cancer, ovary cancer, prostate cancer, kidney cancer, bladder cancer, rhabdomyosaroma, fibrosarcoma, osteosarcoma, chondrosarcoma, skin cancer and various types of leukemia (e.g., acute myeloid leukemia, acute lymphatic leukemia, chronic myeloid leukemia, chromic lymphatic leukemia, adult T-cell leukemia and malignant lymphoma). The cancers recited above may be primary, metastatic or complicated with other disease.

When using the pharmaceutical composition of the invention as a gene therapeutic agent, the pharmaceutical composition may be administered directly by injection or by administering a vector integrated with the nucleic acid. Examples of such vector include adenovirus vector, adeno-associated virus vector, herpes virus vector, vaccinia virus vector, retrovirus vector and lentivirus vector. Use of these virus vectors allows efficient administration.

Alternatively, the pharmaceutical composition of the invention may be introduced into a phospholipid vesicle such as liposome for administration. A vesicle carrying siRNA or shRNA is transfected into a predetermined cell by lipofection technique. The obtained cell is systemically administered, for example, intravenously or intraarterially. Local administration into brain or the like is also possible.

A given dose of the pharmaceutical composition of the invention varies depending on age, sex, condition, administration route, number of doses given and dosage form. For example, a dose given using adenovirus is about 10⁶ to 10¹³, once a day for a period of 1 to 8 weeks.

In order to introduce siRNA or shRNA into a tissue or an organ of interest, a commercially available gene transfer kit (e.g., AdenoExpress: Clontech) may be used.

Hereinafter, the present invention will be described more specifically by way of examples. The present invention, however, is not limited to these examples.

### EXAMPLE 1: Examination of p53 activation in MEF cultured cells

p53 in synoviolin homozygous knockout fetal mouse (syno-/-) fibroblasts (MEFs) was detected by western blotting and the cells were further confirmed by immunohistostaining.

Specifically, immunostaining was conducted by fixing MEFs onto a glass slide according to a conventional method and using an anti-p53 antibody (mouse monoclonal antibody BD: Becton, Dickinson). A specimen that was blocked with 3% bovine serum albumin (BSA) for 30 minutes was immunoreacted with the anti-p53 antibody diluted with 0.3% BSA (BD: 10 µg/ml) at room temperature for 60 minutes. The reacted specimen was washed with PBS, and immunoreacted with TRITC-labeled anti-mouse IgG antibody (Dako) as a secondary antibody. Antigens that immunoreacted with the anti-p53 antibody were confirmed under a fluorescent microscope.

As a result, a higher number of cells resulting p53 activation were confirmed in the syno-/- MEF cultured cells as compared to that in the wild type (Figure 1: panel "MEF-/-").

### EXAMPLE 2: Examination of p53 activation in syno-/- mouse

### p53 activation in syno-/- mouse was examined by immunostaining using an embryo.

Specifically, immunostaining of syno-/- fetal mouse was conducted by fixing a tissue on a glass slide according to a conventional method and using VECTASTAIN ABC kit (VECTOR). A specimen that was blocked with a blocking reagent for 30 minutes was immunoreacted with anti-p53 antibody FL393 diluted to 5 µg/ml at room temperature for 60 minutes. The reacted specimen was washed with PBS, and immunoreacted with HRP-labeled anti-rabbit IgG antibody as a secondary antibody. Antigens that immunoreacted with the anti-p53 antibody were confirmed by color development of 3,3'-diaminobenzidine tetrahydrochloride based on HRP activity. Methyl green staining was performed for comparison.

As a result, p53 activation was confirmed in the syno-/- embryo (Figure 2).

### EXAMPLE 3: Effect of synoviolin on p53

p53 in syno-/- MEF cultured cells was detected by western blotting.

Specifically, a disrupted cell fraction was prepared for each type of cells by using a cell disrupting agent (50 mM Tris-HCl (pH 8.0), 150 mM NaCl, 1% NP40, 1 mM PMSF, 0.1% sodium dodecyl sulfate (SDS), 2 µg/ml Leupeptin, 2 µg/ml Aprotinin and 2 µg/ml Pepstatin). Then, the disrupted cell fractions were separated by SDS polyacrylamide electrophoresis (SDS-PAGE). Following SDS-PAGE, the cell-derived proteins were transferred onto a nitrocellulose (NC) membrane by an electroblotting technique. After blocking this NC membrane with Tris buffered saline (TBS) supplemented with 5% skimmed milk at room temperature for an hour, it was immunoreacted with anti-p53 antibody c-terminal aa; 195-393 or FL393 diluted with TBS supplemented with 5% skimmed milk at room temperature for an hour. The reacted NC membrane was washed with 0.1% Tween20/TBS, immunoreacted with horse radish peroxidase (HRP)-labeled anti-rabbit IgG antibody as a secondary antibody at room temperature for an hour and washed with 0.1% Tween20/TBS. HRP activity was detected to detect antigens of interest. HRP activity was detected using ECL kit (Amersham) (Clinical Chemistry. 25, p1531, 1979).

As a result, increase in p53 expression level was confirmed in syno-/- MEF cultured cells by western blotting (Figure 3).

### EXAMPLE 4: Identification of phosphorylated site of p53 in syno-/- MEF cultured cells

In this example, a phosphorylated site of p53 was identified by western blotting using an anti-p53 antibody.

Specifically, four types of anti-phosphorylated-p53 monoclonal antibodies that recognize phosphorylations of different serine residues of p53 (SEQ ID NO:17) (Phospho-p53(ser15), Phospho-p53(ser20), Phospho-p53(ser37) and Phospho-p53(ser46); Becton, Dickinson) were used for western blotting method following SDS-PAGE separation of the MEF cell protein. Western blotting method was carried out as described in Example 3 except that the anti-phosphorylated-p53 monoclonal antibodies were used as primary antibodies and anti-mouse IgG sheep-HRP was used as a labeled antibody.

As a result, phosphorylation of the serine residue at position 15 was notable in the p53 amino acid sequence (SEQ ID NO:17) in syno-/- MEF cultured cells (Figure 4). In Figure 4, the left upper panels show the phosphorylated serine residue at position 15. A strong band can be seen around 53 kDa.

### EXAMPLE 5: Understanding the mechanism of Ser15 phosphorylation enhancement

RKO cell line (human colorectal cancer-derived cell line) confirmed to be expressing wild-type p53 was seeded at 1.0 x 10⁵ cells/plate/2 mL on a 60-mm plate. Seventy-two hours following transfection of siRNAs targeting GFP and synoviolin using Oligofectamine, caffeine (10 mM) as an inhibitor of ATM (ataxia-telangiectasia mutated) and ATR (ATM and Rad3 related) that are critical for Ser15 phosphorylation was added. Western blotting was performed using antibody Phospho-p53(ser15) for phosphorylated Ser15-p53.

As a result, Ser15 phosphorylation that was enhanced with siRNA targeting synoviolin was completely inhibited by addition of caffeine (12 and 24 hours after addition) (Figure 5). This indicates that synoviolin generally suppresses p53 by inhibiting the functions of ATM and ATR.

### EXAMPLE 6: Effect of synoviolin on p21 expression induced by p53

RKO cells were subjected to siRNA treatment targeting synoviolin to examine changes in expression of p21 (i.e., transcript of p53) by western blotting.

Specifically, an anti-p21 polyclonal antibody (Santa Cruz) was used. RKO cell line (human colorectal cancer-derived cell line) confirmed of expressing wild-type p53 was seeded at 1.0 x 10⁵ cells/plate/2 mL on a 60-mm plate. Seventy-two hours following transfection of siRNAs targeting GFP and synoviolin using Oligofectamine, proteins were separated by SDS-PAGE, followed by western blotting method. Western blotting method was carried out as described in Example 3 except that the anti-p21 polyclonal antibody was used as a primary antibody and anti-mouse IgG sheep-HRP was used as a labeled antibody.

As a result, siRNA treatment targeting synoviolin enhanced p53 expression as well as p21 expression. This effect was clearly observed by 72 hours (Figure 6).

### EXAMPLE 7: Examination of effect of inhibition of synoviolin expression on expression of p53-associated protein, cell cycle and the like

In this example, effects of synoviolin inhibition in synovial cells by RNAi effect on expression of p53-associated protein and cell cycle were examined.

RA synovial cells were seeded on a 10-cm dish at 9.0 x 10⁴cells, transfected with synoviolin siRNA (final concentration 25 nM), and the cell cycle was observed with a flow cytometer. As a result, a delay was observed in G0/G1 cell cycle phases with 25 nM siRNA (No. 589) (Figure 7).

As siRNA, h589 was used.

Here, h589 refers to a double-stranded RNA formed by annealing the following sense and antisense strands.

Sense strand h589: GGU GUU CUU UGG GCAACU G TT (SEQ ID NO:18)

Antisense strand h589: CAG UUG CCC AAA GAA CAC C TT (SEQ ID NO:19)

### EXAMPLE 8: Examination of synoviolin expression in cancer tissues

Tissue arrays (CHEMICON: 10 common human cancer tissues with normal human tissues) were subjected to immunostaining using an anti-synoviolin antibody (10 Da).

The concentration of antibody used for immunostaining was 8 µg/ml and Simplestain MAX (M) kit was used.

As a result, synoviolin expressions in normal tissues were observed in large intestine, kidney, lung, ovary, testis, skin and mammary gland whereas no expression was observed in nerve and lymph node. Moreover, synoviolin expression was confirmed in each of the tumor tissues. Especially, expressions were clearly enhanced in nerve and lymph node (Figures 8 and 9).

### EXAMPLE 9: Effect of co-expression of synoviolin and p53 in cultured cells on localizations thereof

Three types of plasmids, GFP-p53, FLAG-synoviolin and FLAG-synoviolin C307S (without ubiquitination (Ub) activity) were introduced into Saos-2 cells.

Each of the plasmids was as follows.

GFP-p53: Expression of a fusion protein of green fluorescence protein fused to wild-type p53.

FLAG-synoviolin: Expression of FLAG-tagged wild-type synoviolin.

FLAG-synoviolin C307S (no ubiquitination (Ub) activity): Expression of FLAG-tagged deactivated synoviolin.

Twenty-four hours following transfection with FuGene6(Roche), cells were fixed in 10% formalin, and the nuclei were stained with 400-fold diluted primary antibody α-FLAG antibody, 200-fold diluted secondary antibody α-mouse IgG-TRITC and 1 µM DAPI to observe their localizations.

As a result, when wild-type p53 was strongly expressed, it was found localized to the nucleus (Figure 10). When wild-type synoviolin was strongly expressed, it was localized to the cytoplasm (especially around the nucleus). When wild-type p53 and wild-type synoviolin were co-expressing, p53 that is normally localized to the nucleus was distributed in a small dot pattern around the nucleus and co-localized with synoviolin (Figure 11). When wild-type p53 and synoviolin C307S mutant were co-expressing, p53 formed a large dot in the cytoplasm and co-localized with synoviolin (Figure 12).

These results indicate that synoviolin and p53 co-localize under certain conditions. The localization patterns seem to change depending on the presence of ubiquitination activity.

### EXAMPLE 10: Examination of in vitro ubiquitination of GST-p53 with MBP-synoviolin dTM-His

The amount of protein p53 in a cell has been observed to fluctuate depending on increase and decrease in the amount of synoviolin in the cell, suggesting synoviolin's control over p53. In order to examine whether synoviolin directly ubiquitinates (Ub) p53, *in vitro* Ub reaction was examined using GST-p53 and MBP-synoviolin dTM-His.

GST-p53: A fraction obtained by expressing p53 fused to GST at the N-terminal end in *E. coli* and purifying it.

MBP-synoviolin dTM-His: A fraction obtained by expressing synoviolin fused to MBP tag at the N-terminal end and His tag at the C-terminal end in *E. coli* and purifying it.

*E. coli* (BL21) carrying pGEX/p53 was grown in 500 ml LB medium. Following induction with IPTG (1 mM, 30°C, 6h), *E. coli* extract was prepared from the culture solution by using a buffer containing 0.5% NP-40.

GST-p53 was purified from the *E*. *coli* extract using a GSH-sepharose resin in the presence of 0.1% NP-40. The dialyzed sample was used for reaction in combination with MBP-synoviolin dTM-His and other compositions used in *in vitro* Ub reaction (ATP, PK-His-HA-Ub, yeast E1, His-UbcH5c) (Figure 13). After the reaction, the proteins were separated by 7.5% SDS-PAGE and transferred onto a PVDF membrane to detect protein p53 on the membrane with an anti-p53 antibody (FL393 or DO-1). The same reaction and detection were conducted by changing the loadings of GST p53.

As a result, when purified GST-p53 fraction and all of the compositions including MBP-synoviolin dTM-His were added, a ladder-like signal from p53 was observed centering around about 90 kDa (Figure 13). These signals enhanced in a GST-p53-loadings-dependant manner. These results suggest that synoviolin is directly involved in p53 ubiquitination, thus indicating that inhibition of synoviolin expression and/or function can suppress p53 ubiquitination.

### EXAMPLE 11: Examination of amounts of synoviolin and p53 mRNAs under RNAi

In this example, changes in mRNA amounts were examined for synoviolin and related genes in time course under synoviolin RNAi conditions to examine the effects of synoviolin on cell cycle and apoptosis.

RA synovial cells were seeded at 30,000 cells/10 cm-dish, and transfected with 25 nM siRNA (No. 589) according to a conventional method. Cells were collected in a time-course-manner during 4 days of cell cultivation to obtain mRNAs. Reverse transcription PCR was performed using 1 µg mRNA as a temperate together with random primers to obtain cDNA. The obtained cDNAs were quantitated using ABI TaqMan Gene expression assay (GEX). The amount of mRNA was calculated using 18S rRNA as the control gene.

GEX reagent target assay Nos. (assay IDs) Hs00381211_ml and Hs00153340_ml were assigned to synoviolin and TP53, respectively.

As a result, the amount of synoviolin mRNA decreased in the presence of synoviolin siRNA while the amount of p53 mRNA showed no change (Figure 14).

### EXAMPLE 12: Determination of p53-binding domain of synoviolin

A necessary and sufficient condition for GST-synoviolin to bind to p53 in *in vitro* pulldown assay is the presence of 35 amino acid residues at 236-270 of synoviolin protein amino acid sequence (SEQ ID NO:2).

In this example, synoviolin mutants lacking p53-binding domains were prepared as shown in Figure 15 and GST pulldown assay for ³⁵S-p53 was conducted as described below to further identify a domain necessary for synoviolin to bind to p53 (Figure 16).

A plasmid coding each of the GST proteins (1 µL) was transformed into 100 µL of competent cells (BL-21 strain). The GST proteins and plasmids were as follows.
GST protein: Plasmid
GST: pGEX-6P-1 (Pharmacia Biotech)
GST-SynoΔTM 236-617: pGEX-5-1/SΔTM
GST-SynoΔTM 236-270: p6-3
GST-SynoΔTM 271-617: pST490

Four mL of LB-Amp+ was inoculated and cultured overnight at 37°C. On the following day, OD600 was determined for the precultures, which were used to inoculate 15 ml of LB-Amp+ for OD600 = 3.0 (final concentration ~0.2). After cultivation in a thermostatic bath at 25°C for about 2 hours and confirming that OD600 was 0.6-0.8, the thermostatic bath was cooled down to 20°C with ice. The culture vessel was placed in the bath for 10 minutes and allowed to cool down to 20°C. Fifteen µL of 0.1 M IPTG (final concentration = 0.1 mM, 1/1000 the normal concentration and 150 µL of 1 mM ZnCl₂ (final concentration = 10 µM) were added for shake culture at 20°C for 4 hours to induce expression of GST protein. After the induction, cells were collected by centrifugation (5,000 rpm, 5 min., 4°C). Cells were resuspended in 1 ml PBS(-), and transferred to an Eppendorf tube to collect the cells (14,000 rpm, 1 min., 4°C). After completely taking up the supernatant, the resultant was resuspended in 500 µL PBS(-)/Z (PBS(-)/10 µM ZnCl₂), frozen with liquid nitrogen and stored at -20°C. On the following day, samples at -20°C were melted in a thermostatic bath at 37°C for 10 minutes, and then cooled down to 0°C in an ice water. The following protease inhibitors were mixed and added for 6.5 µL per sample.

| | |
|---|---|
| 100 mM PMSF (Final 1 mM) | 20 µl |
| Aprotinin (Final 0.1 %) | 2 µl |
| 0.5 mg/ml Pepstatin A (Final 0.5 µg/ml) | 2 µl |
| 1 mg/ml Leupeptin (Final 1 µg/ml) | 2 µl |

Each sample was subjected to ultrasonic disruption (power level 7, 15 sec., 3 times). For each time, samples were cooled in ice for 30 seconds. Then, 500 µL of 2 x GST buffer/Z (2% TritonX-100, 720 mM NaCl, 1 x PBS(-), 10 µM ZnCl₂, 10 mM β-mercaptoethanol, 2 mM PMSF, 0.1% aprotinin) was added, mixed and subjected to another ultrasonic disruption (power level 7, 15 sec., once). The solutions subjected to disruption were centrifuged at 14,000 rpm at 4°C for 30 minutes. Meanwhile, 200 µL of 80%-slurry glutathione sepharose beads were washed with 1 ml of 1 x PBS(-) for three times and added with 160 µL of 1 x PBS(-) to prepare 50%-slurry. To 1 ml of the centrifuged supernatant, 80 µL of the 50%-slurry glutathione sepharose beads were added, and the resultant was rotated at 4°C for 2 hours to allow the GST protein to bind to the beads. The beads were washed with 1 mL of 1 x GST-buffer/Z (1% TritonX-100, 360 mM NaCl, 0.5 x PBS(-), 5 µM ZnCl₂, 5 mM β-mercaptoethanol, 1 mM PMSF, 0.05% aprotinin) for four times. Centrifugation was conducted at 2,000 rpm at 4°C for 1 minute. The remaining supernatant was completely taken up, followed by addition of 60 µL of 1 x GST-buffer/Z to amount to a total of 100 µL. Ten µL of the resultant was mixed with an equal amount of 2 x SDS sample buffer, heated with a heat block at 100°C for 5 minutes and applied to 10% gel for 10 µL each. At the same time, 0.25 to 4 µg BSA was also applied. Following electrophoresis (150V, 50 min.), CBB staining (30 min. with fresh staining solution), destaining (1 hr., twice), glycerol water (30-60 min.) and gel drying (80°C, 1 hr.), expressions and recovery efficiencies of the GST proteins were checked. On the following day, ³⁵S-p53 was translated *in vitro.* First, the following reagents were mixed.

| | |
|---|---|
| TNT Reticulocyte Lysate (-80°C) | 25 µL |
| TNT Reticulocyte Buffer (-80°C) | 2 µL |
| Amino acids mixture (-Met) (-80°C) | 1 µL |
| DEPC-treated water | 15 µL |
| RNase inhibitor (cell culture room at -20°C) | 1 µL |
| TNT polymerase (cell culture room at -20°C) | 1 µL |
| ³⁵S-Met | 4 µL |
| Plasmid (p53-HA) | 1 µL |
| Total | 50 µL |

*In vitro* translation took place while keeping the temperature in a thermostatic bath at 30°C for 1.5 to 2.5 hours. Meanwhile, the lid of a G-25 column was loosened and mildly centrifuged (2,500 rpm, 1 min., 4°C), to which 100 µL pulldown buffer V (20 mM HEPES pH 7.9, 150 mM NaCl, 0.2% Triton X-100) was loaded. The column was again centrifuged and washed. To this column, the whole amount (50 µL) of *in vitro* translation solution was loaded and centrifuged (2,500 rpm, 1 min., 4°C). Another 200 µL of pulldown buffer V was loaded and centrifuged (2,500 rpm, 1 min., 4°C). The resultant was used as an *in vitro* translation product (IvTL). Four µL of this product was mixed with 16 µL of Milli-Q and 20 µL of 2 x SDS buffer to prepare an onput of 10%. To 1 ml of pulldown buffer V containing 30 µg of beads binding to GST protein, 120 µL of IvTL was added and rotated at 4°C for an hour. Following centrifugation (10,000 rpm, 1 min, 4°C), 370 µL each of the supernatant was added to each of 1 ml pulldown buffers V containing GST and GST-synoviolin beads and rotated at 4°C for an hour. The beads were washed with 1 ml pulldown buffer V for 4 times. The supernatants were made sure to remain for about 100 µL so as not to take up the beads. Centrifugation took place at 2,500 rpm at 4°C for a minute. The supernatant was taken up and 40 µL of 1 x SDS sample buffer was added to obtain pulldown samples. The 10% onput and pulldown samples were heated at 100°C for 5 minutes and stored at -20°C. On the following day, the samples were warmed in a thermostatic bath at 37°C for 10 minutes and 10 µL of each sample was applied on 10% gel. Following electrophoresis (150V, 50 min.), CBB staining (30 min.), destaining (1 hr. x 2), glycerol water (30-60 min.) and gel drying (80°C, 1 hr.), gel was exposed to IP plate. Fourteen hours later, the IP plate was read with BAS, followed by quantitation by ImageGauge. The gel stained with CBB was read with a film scanner.

As a result, the mutants lacking p53-binding domains almost completely lost binding activity. Referring to Figure 15, the 35 amino acids at positions 236-270 seems to be the only p53-binding domain.

### INDUSTRIAL APPLICABILITY

The present invention provides a substance that promotes activity of tumor suppressor gene p53. This substance can activate p53 and allow transportation of p53 to the nucleus and so is useful as a pharmaceutical composition for treating cancer. According to the present invention, cancer can be treated by inhibiting synoviolin expression and/or function.

### SEQUENCE LISTING FREE TEXT

SEQ ID NO: 17: Synthetic oligonucleotide (DNA/RNA mixture).

SEQ ID NO: 18: Synthetic oligonucleotide (DNA/RNA mixture).

## Claims

1. A pharmaceutical composition comprising a substance that promotes activity of tumor suppressor gene p53 or protein p53.

2. A pharmaceutical composition according to claim 1, wherein the substance that promotes activation of tumor suppressor gene p53 or protein p53 is a substance that inhibits synoviolin expression and/or function.

3. A pharmaceutical composition according to claim 2, wherein the substance that inhibits synoviolin expression and/or function is siRNA or shRNA that targets a gene coding for synoviolin.

4. A pharmaceutical composition according to claim 3, wherein the gene coding for synoviolin comprises the nucleotide sequence represented by SEQ ID NO:1.

5. A pharmaceutical composition according to claim 3, wherein siRNA targets a part of the nucleotide sequence represented by SEQ ID NO:1.

6. A pharmaceutical composition according to any one of claims 1 to 5 for treating cancer.

7. A method for activating tumor suppressor gene p53 or protein p53 comprising inhibiting synoviolin expression and/or function.

8. A method for localizing protein p53 to the nucleus comprising inhibiting synoviolin expression and/or function.

9. A method for suppressing cancer comprising inhibiting synoviolin expression and/or function to localize protein p53 to the nucleus.

10. A method according to claim 9 further comprising irradiating protein p53 localized in the nucleus with radiation or ultraviolet.

11. A method according to claim 9 further comprising contacting a cell containing protein p53 localized to the nucleus with an anticancer agent, or further comprising embolizing a vessel around said cell.

12. A method for phosphorylating a part of amino acid residues of protein p53, comprising inhibiting synoviolin expression and/or function.

13. A method according to claim 12, wherein the part of amino acid residues is serine residue at position 15.

14. A method for activating kinase, comprising inhibiting synoviolin expression and/or function.

15. A method according to claim 14, wherein the kinase comprises ATM, ATR or an enzyme having a similar activity thereto.

16. A method for inducing expression of protein p21 with activated protein p53, comprising inhibiting synoviolin expression and/or function to activate protein p53.

17. A method for suppressing cancer comprising inhibiting synoviolin expression and/or function to allow protein p53 to induce expression of protein p21.

18. A method for activating protein p53, comprising inhibiting synoviolin expression and/or function.

19. A method according to any one of claims 7 to 18, wherein the synoviolin expression is inhibited with siRNA or shRNA that targets a gene coding for synoviolin.

20. A method according to any one of claims 7 to 18, wherein the synoviolin function is inhibited by inhibiting functions of synoviolin to bind to and/or ubiquitinate protein p53.

21. A method according to claim 19, wherein the gene coding for synoviolin comprises the nucleotide sequence represented by SEQ ID NO:1.

22. A method according to claim 19, wherein siRNA targets a part of the nucleotide sequence represented by SEQ ID NO:1.
